Europäisches Patentamt

European Patent Office

Office européen des brevets

(19)

(11) Veröffentlichungsnummer: **0 302 440 B1**

# EUROPÄISCHE PATENTSCHRIFT

(12)

(45) Veröffentlichungstag der Patentschrift: **28.10.92**

(51) Int. Cl.$^5$: **C07D 295/02**

(21) Anmeldenummer: **88112507.4**

(22) Anmeldetag: **02.08.88**

(54) Verfahren zur Gewinnung von N-Ethylpiperazin.

(30) Priorität: **05.08.87 DE 3725925**

(43) Veröffentlichungstag der Anmeldung:
**08.02.89 Patentblatt 89/06**

(45) Bekanntmachung des Hinweises auf die
Patenterteilung:
**28.10.92 Patentblatt 92/44**

(84) Benannte Vertragsstaaten:
**BE DE ES FR GB IT NL**

(56) Entgegenhaltungen:
**EP-A- 0 181 536**
**FR-A- 1 592 964**

(73) Patentinhaber: **BASF Aktiengesellschaft
Carl-Bosch-Strasse 38
W-6700 Ludwigshafen(DE)**

(72) Erfinder: **Müller, Herbert, Dr.
Carostrasse 53
W-6710 Frankenthal(DE)**
Erfinder: **Franz, Dieter, Dr.
Horst-Schork-Strasse 75
W-6700 Ludwigshafen(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 302 440 B1

## Beschreibung

Die vorliegende Erfindung betrifft ein neues Verfahren zur destillativen Gewinnung von N-Ethylpiperazin aus seinen Gemischen mit Piperazin, N,N'-Diethylpiperazin, Wasser und untergeordneten Mengen Ethanol und sonstigen Bestandteilen.

N-Ethylpiperazin ist ein wichtiges Zwischenprodukt für organische Synthesen, das in der Technik bekanntermaßen nach verschiedenen Verfahrensvarianten aus Ethanol und Piperazin hergestellt wird. Hierbei erhält man regelmäßig Gemische, die neben dem N-Ethylpiperazin das weniger erwünschte N,N-Diethylpiperazin, nicht umgesetztes Piperazin, überschüssiges Ethanol, das bei der Reaktion entstehende Wasser sowie geringe Mengen einiger Nebenprodukte enthalten.

Wie weiterhin allgemein bekannt ist und aus der EP-A-181 536 ausdrücklich hervorgeht, bereitet die destillative Aufarbeitung dieser Gemische erhebliche Schwierigkeiten, die vor allem auf die Anwesenheit des Piperazins und des Wassers zurückzuführen sind.

Nach der Lehre dieser EP-A umgeht man diese Schwierigkeiten, indem man die Reaktion unter bestimmten Bedingungen in Gegenwart relativ großer Mengen Wasser vornimmt, wodurch es ermöglicht wird, das Piperazin nahezu vollständig umzusetzen. Die anschließende destillative Aufarbeitung gestaltet sich dann wesentlich einfacher, weil mehrere Piperazin-Azeotrope entfallen.

Allerdings vermag auch dieses Verfahren nicht voll zu befriedigen, weil der vollständige Piperazinumsatz eine Selektivitätsverminderung bezüglich des N-Ethylpiperazins bedingt und weil man infolgedessen die Bildung erheblicher Mengen des weniger erwünschten N,N'-Diethylpiperazins in Kauf nehmen muß.

Der Erfindung lag daher die Aufgabe zugrunde, diesen Nachteilen, nämlich den Aufarbeitungsschwierigkeiten einerseits und der unzureichenden Selektivität bezüglich des N-Ethylpiperazins andererseits, gleichermaßen abzuhelfen.

Demgemäß wurde ein Verfahren zur destillativen Gewinnung von N-Ethylpiperazin aus Gemischen gefunden, die pro 100 kg aus

a = 30 bis 90 kg N-Ethylpiperazin
b = 7 bis 40 kg N,N'-Diethylpiperazin
c = 3 bis 20 kg Piperazin
d = 0 bis 30 kg Ethanol
e = 0 bis 40 kg Wasser sowie
f = 0 bis 5 kg Begleitstoffen

bestehen, welches dadurch gekennzeichnet ist, daß man die Destillation in Gegenwart von

$$x = y + e = 3b \text{ bis } 12b$$

Wasser vornimmt, wobei y diejenige Wassermenge ist, die zur Erfüllung dieser Bedingung gegebenenfalls zuzufügen ist, und daß man von diesen Gemischen nacheinander die Fraktionen

1. Wasser, Ethanol, N,N'-Diethylpiperazin und leichtflüchtige Begleitstoffe,
2. Wasser, falls es im Überschuß vorliegt,
3. Piperazin und
4. reines N-Ethylpiperazin

als Kopfprodukte abtrennt.

Dieses Verfahren beruht auf der überraschenden Beobachtung, daß das N,N'-Diethylpiperazin aus den Gemischen der angegebenen Zusammensetzung zusammen mit dem Wasser eine unter 100°C siedende azeotropartige Fraktion bildet und auf diese Weise leicht abgetrennt werden kann, obwohl es selber höher siedet (172°C) als Piperazin (147°C) und N-Ethylpiperazin (156°C).

Die Ausgangsgemische fallen bei der in verschiedenen Verfahrensvarianten bekannten Ethylierung von Piperazin mit Ethanol an. Meist nimmt man diese Reaktion in Gegenwart von Hydrierkatalysatoren und Wasserstoff, häufig auch in Gegenwart von Wasser und Mineralbasen wie Natriumcarbonat oder Calciumhydroxid vor, wobei man das Ethanol zweckmäßigerweise im Überschuß über das Piperazin einsetzt.

Nach Abtrennung des festen Hydrierkatalysators destilliert man zunächst soviel des überschüssigen Ethanols zusammen mit geringen Mengen Wasser ab, bis eine Ethanol-Konzentration d erreicht ist. Die so erhaltenen Gemische entsprechen den Ausgangsgemischen im vorliegenden Verfahren.

Je nach der Art der vorangegangenen Ethylierung enthalten die Ausgangsgemische entweder praktisch kein Wasser, lediglich das bei der Ethylierung entstehende Reaktionswasser oder, entsprechend der Lehre der EP-A- 181 536, zum Zwecke der Selektivitätsverbesserung zusätzlich in die Ethylierungsreaktion eingeführtes Wasser.

Grundsätzlich ist die Ethylierung nach der EP-A 181 536 zu bevorzugen, allerdings mit dem Unterschied, daß nicht das gesamte Piperazin umgesetzt wird, sondern nur ein Teil, wie er der Menge c im erfindungsgemäßen Ausgangsgemisch entspricht.

Die Menge x des insgesamt bei der Destillation mitzuverwendenden Wassers, die sich aus dem gegebenenfalls zuzusetzenden Anteil y und der bereits vorhandenen Wassermenge e zusammensetzt, hängt definitionsgemäß von der Menge b des N,N'-Diethylpiperazins ab, wobei x = 3b die für die Abtrennung des N,N'-Diethylpiperazins erforderliche Mindestmenge ist. Im allgemeinen empfiehlt es sich jedoch, etwas mehr Wasser zu verwenden - etwa 4b bis 10b -, da die das Wasser und das N,N'-Diethylpiperazin bildende Fraktion keinem Azeotrop konstanter Zusammensetzung entspricht, sondern lediglich azeotropähnliches Verhalten zeigt. Je nach dem Ethanol-Restgehalt d sowie je nach der Art und der Menge der sonstigen Begleitstoffe ändert sich der Wasserbedarf x etwas, so daß ein mäßiger Überschuß weit weniger nachteilig ist als eine nicht voll ausreichende Menge. Lediglich größere Wassermengen als x = 12b lassen keinen Vorteil mehr erkennen.

Man kann dem Ausgangsgemisch zu Beginn der Destillation die gesamte gegebenenfalls erforderliche Wassermenge y zusetzen, jedoch ist es in der Regel zweckmäßiger, das Wasser allmählich in dem Maße zulaufen zu lassen, wie es zusammen mit N,N′-Diethylpiperazin abdestilliert.

Im ersten Destillationsschritt, der bei ungefähr 80°C beginnt, fällt zunächst ein Vorlauf aus Ethanol, Wasser, dem N,N′-Diethylpiperazin und einigen von der Synthese stammenden leichtsiedenden Begleitstoffen wie N-Ethylethylendiamin und N,N′-Diethylethylendiamin an.

Man erhält sodann zwischen etwa 80 und 99°C unter stetigem Ansteigen des Siedepunktes eine azeotrop-ähnliche Fraktion aus Wasser und N,N′-Diethylpiperazin, in welcher der Wasseranteil allmählich zunimmt.

Wird Wasser im Überschuß über die zur Entfernung des N,N′-Diethylpiperazins erforderlichen Menge verwendet, erhält man bei etwa 100°C in einem zweiten Destillationsschritt eine zweite Fraktion, die im wesentlichen aus Wasser besteht.

Im dritten Destillationsschritt fällt bei 147°C das Piperazin als Kopffraktion an und im vierten erhält man bei 156°C das reine N-Ethylpiperazin. Zurück bleibt eine Sumpffraktion, die einige höhersiedende Bestandteile enthält. Das Piperazin kann in die Synthese zurückgeführt werden, und das N,N′-Diethylpiperazin läßt sich durch Extraktion mit Kohlenwasserstoffen oder mittels konzentrierter Natronlauge vom Wasser abtrennen.

Die zur Trennung der vier Fraktionen erforderliche Anzahl an theoretischen Böden liegt zweckmäßigerweise zwischen 15 und 40, vorzugsweise zwischen 20 und 30, und als Rücklaufverhältnisse in den vier Schritten empfehlen sich Werte von 2:1 bis 20:1 (1. Schritt), 2:1 bis 40:1 (2. Schritt), 2:1 bis 20:1 (3. Schritt) und 2:1 bis 20:1 (4. Schritt).

Alle vorstehenden Angaben gelten für das Arbeiten unter Normaldruck (~1 bar), jedoch kann man die Destillation auch ohne weiteres bei vermindertem oder erhöhtem Druck, etwa im Bereich von 0,05 bis 10 bar. ausführen, da sich das Siedeverhalten der Ausgangsgemische sowie der zwischenzeitlich hieraus entstehenden Gemische in diesem Bereich nicht grundsätzlich ändert. Für das Arbeiten unter anderem Druck als Normaldruck wären lediglich die jeweils speziellen Destillationsbedingungen wie Temperaturen, Anzahl der theoretischen Böden und Rücklaufverhältnisse routinemäßig zu ermitteln.

Man kann das erfindungsgemäße Verfahren auch kontinuierlich gestalten, indem man die vier Destillationsschritte wie üblich auf vier Kolonnen verteilt, wobei die für das Arbeiten unter Normaldruck erforderliche Anzahl der theoretischen Böden im Verstärkungsteil für den ersten Schritt etwa 10 bis 15, für den zweiten etwa 20 bis 25, für den dritten etwa 20 bis 25 und für den vierten etwa 5 bis 10 beträgt. Es reicht aus, den Abtriebsteil der vier Kolonnen jeweils mit ca. 10 theoretischen Böden auszustatten.

Apparativ bietet das erfindungsgemäße Verfahren keine Probleme, so daß man es wie üblich in Kolonnen beliebiger Bauart, darunter aus wirtschaftlichen Gründen vorzugsweise in Füllkörperkolonnen, ausführen kann.

Das erfindungsgemäße Destillationsverfahren ist von der Provenienz der Ausgangsgemische nicht erkennbar abhängig, jedoch empfiehlt sich im allgemeinen deren Herstellung nach dem Verfahren der EP-A 181 536, allerdings mit dem Unterschied, daß man das Piperazin zur Selektivitätsverbesserung bezüglich des N-Ethylpiperazins in größeren Mengen einsetzt - Molverhältnis Piperazin/Ethanol etwa 0,6:1 bis 3:1 - und/oder daß man die Synthese nicht soweit führt, bis das Piperazin verbraucht oder praktisch verbraucht ist.

Da mit dem erfindungsgemäßen Verfahren die Aufgabe erfolgreich gelöst wird, Piperazin enthaltende Reaktionsgemische zu fraktionieren und das N-Ethylpiperazin in reiner Form zu gewinnen, ist es nicht mehr erforderlich, das Piperazin weitgehend abreagieren zu lassen, wodurch sich der Anteil des unerwünschten N,N′-Diethylpiperazins auf ein wirtschaftliches Minimum herabsetzen läßt.

Beispiel

1000 g eines Gemisches, welches aus

| | | |
|---|---|---|
| a = | 370 g | N-Ethylpiperazin |
| b = | 120 g | N,N′-Diethylpiperazin |
| c = | 60 g | Piperazin |
| d = | 60 g | Ethanol |
| e = | 370 g | Wasser und |
| f = | 20 g | eines Gemisches aus N,N′-Diethylethylendiamin und N,N,N′-Triethylethylendiamin |

bestand, wurde mit y = 600 g Wasser versetzt (x = y + e = 8,1 b) und in einer Füllkörperkolonne mit etwa 20 theoretischen Böden bei Normaldruck der fraktionierten Destillation unterworfen.

Hierbei wurden bei einem gleichbleibenden Rücklaufverhältnis von 3:1 folgende Fraktionen erhalten:

1. zwischen 78 und 100°C 655 g einer Fraktion mit <0,1 Gew.% N-Ethylpiperazin, 18 Gew.% N,N′-Diethylpiperazin, 9 Gew.% Ethanol, 70 Gew.% Wasser und 3 Gew.% der Begleitstoffe;
2. bei etwa 100°C 325 g einer im wesentlichen aus Wasser bestehenden Fraktion;
3. bei 147°C 60 g Piperazin und
4. bei 156°C 340 g N-Ethylpiperazin.

Etwa 30 g im Kolonnensumpf verbliebener Rückstand entsprechend dem sogenannten "Hold up", bestanden im wesentlichen aus N-Ethylpiperazin.

Die Destillationsausbeute an N-Ethylpiperazin betrug somit 92 %. Berücksichtigt man den in der Kolonne verbliebenden Teil ("Hold-up"), der über mehrere aufeinanderfolgende Destillationen rechnerisch gegen null strebt, entspricht dies einer praktisch quantitativen Ausbeute.

Das Ausgangsgemisch wurde durch Umsetzung von 351 g (4,5 mol) Piprazin und 414 g (9 mol) Ethanol bei 175°C und unter einem Wasserstoffdruck von 60 bar in Gegenwart von 175 g Raney-Nickel und von 290 g Wasser und anschließende Entfernung des Großteils des überschüssigen Ethanols hergestellt.

**Patentansprüche**

1. Verfahren zur destillativen Gewinnung von N-Ethylpiperazin aus Gemischen, die pro 100 kg aus
   a = 30 bis 90 kg N-Ethylpiperazin
   b = 7 bis 40 kg N,N′-Diethylpiperazin
   c = 3 bis 20 kg Piperazin
   d = 0 bis 30 kg Ethanol
   e = 0 bis 40 kg Wasser sowie
   f = 0 bis 5 kg Begleitstoffen
   bestehen, dadurch gekennzeichnet, daß man die Destillation in Gegenwart von

   x = y + e = 3b bis 12b

   Wasser vornimmt, wobei y diejenige Wassermenge ist, die zur Erfüllung dieser Bedingungen gegebenenfalls zuzufügen ist, und daß man von diesen Gemischen nacheinander die Fraktionen
   1. Wasser, Ethanol, N,N′-Diethylpiperazin und sonstige leichtflüchtige Begleitstoffe,
   2. Wasser, falls es im Überschuß vorliegt,
   3. Piperazin und
   4. reines N-Ethylpiperazin
   als Kopfprodukte abtrennt.

**Claims**

1. A process for the distillative isolation of N-ethylpiperazine from a mixture consisting per 100 kg of

   a = from 30 to 90 kg of N-ethylpiperazine
   b = from 7 to 40 kg of N,N'-diethylpiperazine
   c = from 3 to 20 kg of piperazine
   d = from 0 to 30 kg of ethanol
   e = from 0 to 40 kg of water and
   f = from 0 to 5 kg of concomitants,
   which comprises performing the distillation in the presence of

   x = y + e = from 3b to 12b

   of water, y being the quantity of water which has to be added, if necessary, to meet this condition, and successively separating from this mixture the fractions
   1. water, ethanol, N,N'-diethylpiperasine and other highly volatile concomitants,
   2. water, if present in excess,
   3. piperazine and 4. pure N-ethylpiperazine as overhead products.

**Revendications**

1. Procédé de récupération par distillation de N-éthylpipérazine dans des mélanges qui se composant, pour 100 kg, de
   a = 30 à 90 kg de N-éthylpipérazine,
   b = 7 à 40 kg de N,N'-diéthylpipérazine,
   c = 3 à 20 kg de pipérazine,
   d = 0 à 30 kg d'éthanol,
   e = 0 à 40 kg d'eau et
   f = 0 à 5 kg d'impuretés,
   caractérisé en ce qu'on procède à la distillation en présence de

   x = y + e = 3b à 12b

   d'eau, y étant la quantité d'eau qui doit être éventuellement ajoutée pour remplir ces conditions, et en ce qu'on sépare successivement de ces mélanges, en tant que produits de tête, les fractions suivantes
   1. eau, éthanol, N,N'-diétliylpipérazine et autres impuretés très volatiles,
   2. eau, au cas où elle est présente en excès,
   3. pipérazine et
   4. N-éthylpipérazine pure.